**Europäisches Patentamt**

**European Patent Office**

**Office européen des brevets**

(19)

(11) Publication number: **0 397 948**
**A1**

(12) # EUROPEAN PATENT APPLICATION

(21) Application number: **89500059.4**

(22) Date of filing: **19.05.89**

(51) Int. Cl.5: **A61M 5/50, A61M 5/32**

(43) Date of publication of application:
**22.11.90 Bulletin 90/47**

(84) Designated Contracting States:
**AT BE CH DE FR GB GR IT LI LU NL SE**

(71) Applicant: **Martinez Gimeno, Carlos V.**
**Avenida Estacion, 27**
**E-03003 Alicante(ES)**

(72) Inventor: **Martinez Gimeno, Carlos V.**
**Avenida Estacion, 27**
**E-03003 Alicante(ES)**

(74) Representative: **De Arpe Fernandez, Manuel**
**ARPE Patentes y Marcas Guzmán el Bueno,**
**133**
**E-28003 Madrid(ES)**

(54) **Auto-destructable syringe.**

(57) The syringe has a plunger (9; 24; 39) with a breaking point (18; 32), which is broken by being subjeted to rotational pressure, when plunger tip (11; 30) reaches the end of the outer tube (2; 23; 37) of the syringe and all the liquid has been expolled, making the syringe unusable after a single injection. To achieve this, the plunger is equipped with a rotating mechanism (7-13; 26-28; 38-41) at it's lower end. It is also equipped with a gears or transmission system (14-15; 34-35; 42-43) consisting of locking sets of "teeth" which separate the rotating mechanism from the rotational block mechanism so that torque is not produced.

FIG. 6

FIG. 5

FIG. 1

EP 0 397 948 A1

# AUTO-DESTRUCTABLE SYRINGE

The invention consists of a single-injection syringe and more specifically, an auto-destructable syringe, as repeated use is impossible.

Low-cost, disposable, plastic syringes made of polyprppylene or similar materials are widely used and have replaced the older glass syringes with the result that the problems of sterilization produced by glass syringes have been eliminated.

Plastic syringes, nevertheless, can be re-used even though they are no longer sterile, and whether they are thrown away or not after use depends on the user. They are not, therefore, strictly speaking single injection syringes, as they can be re-used by undiscriminating individuals.

The widespread health problems presented by the spreading of infections diseases through re-use of syringes are well-known. The increase in the number of people whith infectious diseases such as viral hepatitis and Aquired Immunodeficiency Syndrome (AIDS) who re-use syringes is especially worrying. This means of infection, however, is not restricted to individuals who re-use syringes. In fact, among professionals working in the Health Service, accidental pricks, or injuries from syringes which have been used on patients are common.

As far as protection from accidental cuts etc. is concerned, present methods are effective. The syringes come with needle covers to protect against injuries but, in fact, this protective capsule ensures that the needle remains sterile while the injection is being prepared and does not therefore prevent injuries of this type.

The idea behind the invention is to produce a lowcost, disposable syringe which is rendered useless during the injection sonthat it cannot be used again. In this way, the syringe is single- injection in the strictest sense.

In addition, a syringe of this type with a protective cover for the needle would be developed, so that the risk of accidental injuries would be reduced or eliminated.

These aims are achieved in a syringe for medical use, with a compartmented plunger tipand outer tube and a rotating plumger with a breaking point near the lower end of the plunger.

Other characteristics include:
- Various rotating mechanisms placed in order to link up in the aforementioned areas of the plunger tip and outer tube of the syringe, so that as the plunger tip is pushed further in, in the course of the injection, the areas mentioned rotate accordingly.
- Various mechanisms for blocking the rotation of the plunger, to be placed in order to link up on the inside of the relevant section of the outer tube and/or in the area of the plunger tip, and which impede the joint rotation of the two sections of the plunger situated on either side of the breaking point, so that as the plunger tip is pushed when injection is taking place, a torque is produced between the two sections of plunger, which is sufficient to break the plunger at the breaking point.
- Varions coupling mechanisms which operate in the same way as gears or a transmission system, located at aither end of the area of the rubber tip which, as the plunger is pulled up, when the liquid is being sucked in, disconnect or uncouple the aforementioned conpling mechanisms, so that a rotational movement is not produced, thereby eliminating a torque and, as a result, avoiding breaking the plunger while the syringe is being filled.

In one version of the invention, the rotational mechanism of the plunger consists of a component which is fitted around the upper part of the plunger. On the outside of the plunger there are rods fixed in the form of a helix and which slot into the grooves on the onter tube, situated on the inside of the syringe. In this version, the blocking mechanisms are rods, situated on the inside of the lower end of the section of the outer tube of the syringe, so that they lie next to the end section of the plunger, or plunger tip, which is usually made of an elastic material such as rubber, in such a waybthat the rotation of this section of the plunger is impeded as the rubber tip cannot slide over the rods.

In this version, the coupling mechanism consists of "fleam teeth" consisting of indents which are complemented by further sets of teeth in the upper part of the plunger and in the component surrounding it.

As will be easily undertood by technicians, so that the sets of "teeth" or coupling mechanism are disconnected when the plunger is pulled back to draw in liquid, there is slight movement or play between the longitudinal rods on the component surrounding the plunger and the helix-shaped grooves on the inside upper section of the outer tube of they syringe. There is also slingt movement or play between the plunger and it's surrounding component.

In another version, the rotational mechanisms of the plunger are located, by contrast, in the lower section of the syringe and consist of hollew channels forming a helix, located on the inside of the outer tube of the syringe and into which the longitudinal rods in the lower part of the plunger fit. In this case, the rotational block mechanisms consist of grooves forming a helix shape into which longitudinal rods in the lower part of the plunger fit. In this version the coupling mechanism or "fleam teeth" are placed near the end of the plunger.

In another version, the rotational,mechanisms also consist of straight rods on the inside of the syringe deposit which brake the plunger tip.

Also, in this case, the coupling mechanisms which allow the plunger to be moved upwards while liquid is drawn in, consist of a transmission system or "fleam teeth" in the lower part of the plunger.

In another version, the mechanisms to brake or block rotation of the rubber tip of the plunger and adjacent area (just below the weak point of the plunger), are produced by changing the shape of the outer tube of the syringe instead of being round, it is oval or polygonal. The section of plunger which is moved during the injection is made in the same shape so that is fits the outer tube exactly, and cannot rotate. Thus, when rotation occurs (as described above), when loquid is being injected, the weak point of the plunger, situated between the non-rotating rubber tip and the upper part of the plunger, breaks.

Other characteristics and advantages of the invention are explained in more detail in the following description complete with diagrams and applicable to both the versions or models of the syringe described and modifications.

Figure 1 shows a part vertical section of a syringe for medical use manufactured in accordance with the description of the invention.

Figures 2 and 4 show further vertical sections of the syringe.

Figures 5 and 6 are different diagrams in vertical section showing how the syringe as shown in figure 1 works.

Figures 7A, B and C show the separate compo nents of another version of the syringe.

Figures 8 and 9 show details of the syringe shown in figure 7.

Figures 10 and 12 show diagrams of another version of the syringe.

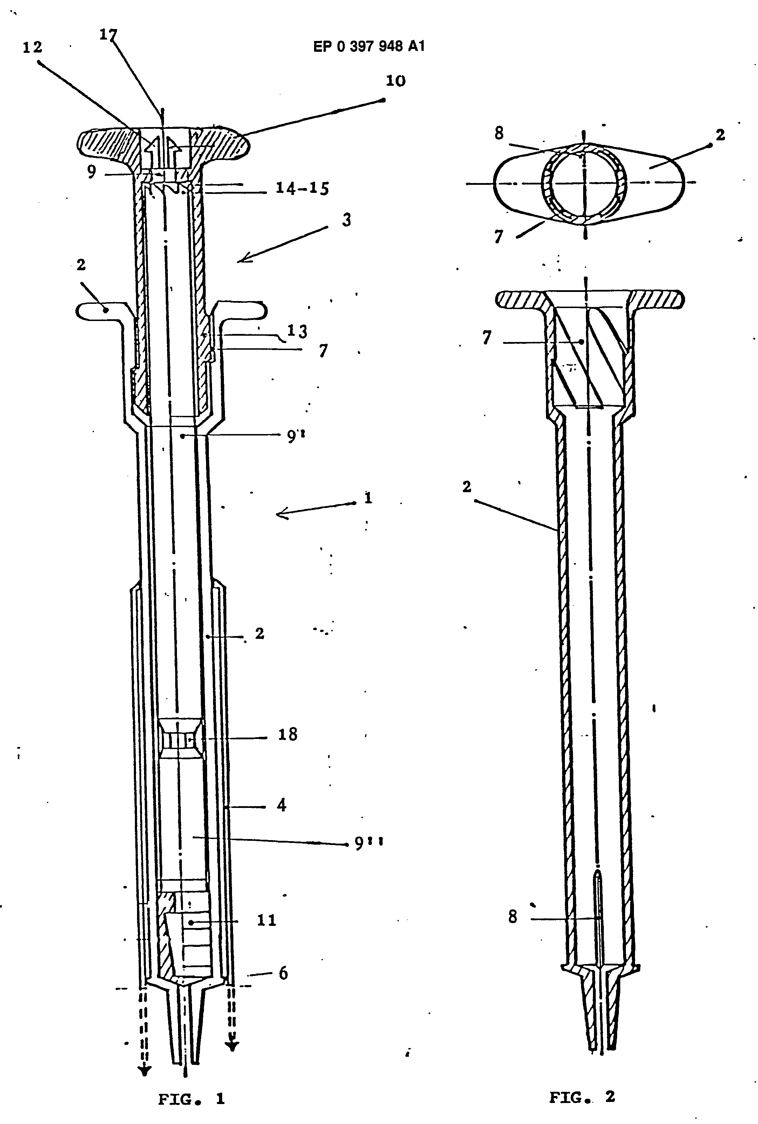

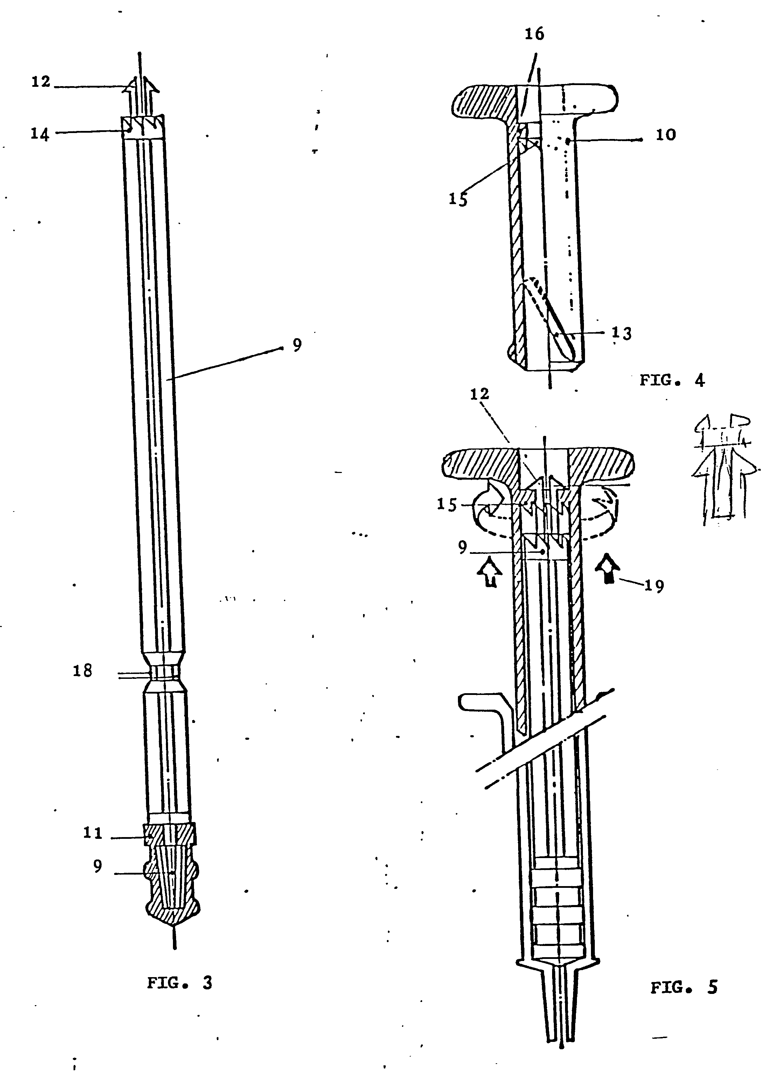

Figures 1 and 4, show a version of the syringe for medical use, with a vertical section of the deposit 2, and the plunger, 3. There is a cylindrical component, 4, around the outside of the outer tube, 2, which slides over the outer tube in the form of a protective cover, so that the needle, 5, can be dept covered by sliding 4 down to the ends, 6, located on the outside of the outer tube of the syringe.

The syringe deposit has, grooves at one end, 7, forming a helix in its inner, upper section and which make a screwlike movement. At the other end of the deposit also on the inside there are several straight rods attached, 8.

The plunger is made up of a tube, 9, and a pushing grip, 10, fitted to one another as described below. The tube, 9, has a rubber or similar tip, 11, on one end, and a plug-like mechanisms, 12, on the other with a slit or gap in the centre. In addition, at the lower end of the plunger is a breaking point (Weak point), 18, so that the plunger is divided into two sections $9'$ and $9''$ on either side of the breaking point. The plunger grip, 10, which fits around the plunger, 9, has rods, 13, on its outer surface which fit into, helix-shaped grooves, 7, located on the aforementioned section of the outer tube of the syringe. In addition, there is a set of 'teeth' or indents, 14, at the upper end of the plunger which fit into another set, 15, on the inside of the plunger grip, 10. The plunger grip, 10, has a cavity, 16, in its upper part for the coupling of the 'plug', 12, of the plunger. In this way the plunger and the plunger grip, 10, are locked together an axle, 17, in a clockwise direction. They unlock when turned in an anti-clock-wise direction.

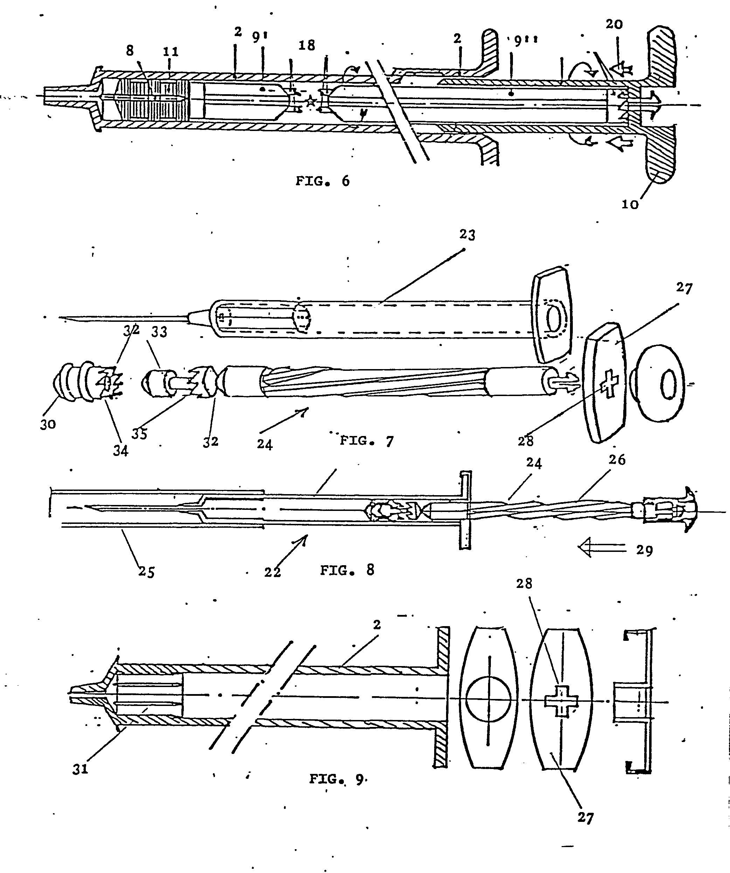

Studying figures 5 and 6 we observe how the syringe in figure 1 works.

Looking especially at figure 5 the plunger grip, 10, is pulled back in the directions of the arrows, 19, to draw in the liquid. During this operation, the plunger is withdrawn whith out any rotational movement, and the liquid is drawn into the outer tube of the syringe.

Looking at figure 6, we observe that during injection, the reverse happens. The plunger grip, 10, is pushed in the direction of the arrows, 20, so that the indents or sets of teeth.

14 and 15 located at the upper end ofthe plunger and inside the plunger grip, 10, are locked together so that the pressure exerted on the plunger grip is transferred to the plunger. If pressure is further exerted in the same direction, the rods, 13, on the plunger grip, 10, are forced into the helix shaped grooves in the outer tube of the syringe producing a joint rotational, clockwise, downwards movement of the plunger and plunger grip, 9 and 10, and a resulting rotational, clockwise, downwards movement of the plunger tip, 11. When pressure is applied in the direction of the arrows, 20, the plunger, 9, and plunger grip, 10, act as a single unit as they are locked together by sets of indents or teeth, 14 and 15, which act as a gears or a transmission mechanism. As more pressure is applied to the plunger grip, and the plunger tip moves further down, it eventually reaches the lower section of the outer tube of the syringe, 2, where the· longitudinal rods, 8, are situated. At this moment, the rotational movement of the tip is blocked or braked as the tip cannot slide over the rods. As the plunger and plunger tip are act as a single unit a torque is produced between sections $9'$, and, $9''$, of the plunger which forces the breaking-point to break. The breaking-point is designed to break when the plunger tip reaches the bottom of the outer tube, so that all the liquid is expelled first.

Figures 7 to 9 represent another version of the invention, the syringe is of a similar design. The outer tube, 23, and plunger tip, 24, are divided into sections. It also has a sliding needle cover or cap which fits onto the outer tube of the syringe.

In this instance, a screw mechanism consisting of a helix-shaped thread, 26, which runs through the middle of the plunger and passes trough a 'guide', 27, with a cross-shaped opening, 28, produces a clockwise, downwards rotating movement. In this way, the plunger tip, 30, which has been locked onto the lower end of the plunger, 24, rotates in a downward movement as the plunger is pushed downwards. This rotational movement is blocked or braked when the plunger tip comes into contact with the rods, located in the end section of the outer tube of the syringe, this, in turn, produces a torque which, when applied to the breaking point, causes it to break. In this version, the resistance of the breaking point, 32, is calculated so that it breaks at exactly the same moment asthe plunger tip reaches the bottom of the outer tube of the syringe and, consequently, after all the liquid has been expelled. Also, in this version, the plunger does not break at the breaking point, 32, on withdrawal, as there is a coupling mechanism between the plunger tip, 30, and the lower end, 33, of the plunger, 24, andplunger tip, 30, are locked together by a coupling mechanism between the lower end, 33, of the plunger 24 and the opening, 34, in the plunger tip, 30. The plunger, 24, and plunger tip, 30, rotate in unison in a clockwise, downwards movement during the injection but rotate independently in an upwards, anticlockwise movement when the plunger is withdraw due to indents or sets of 'teeth', 35 and 36 located at the lower end of the plunger, 24, and on the plunger tip, 30.

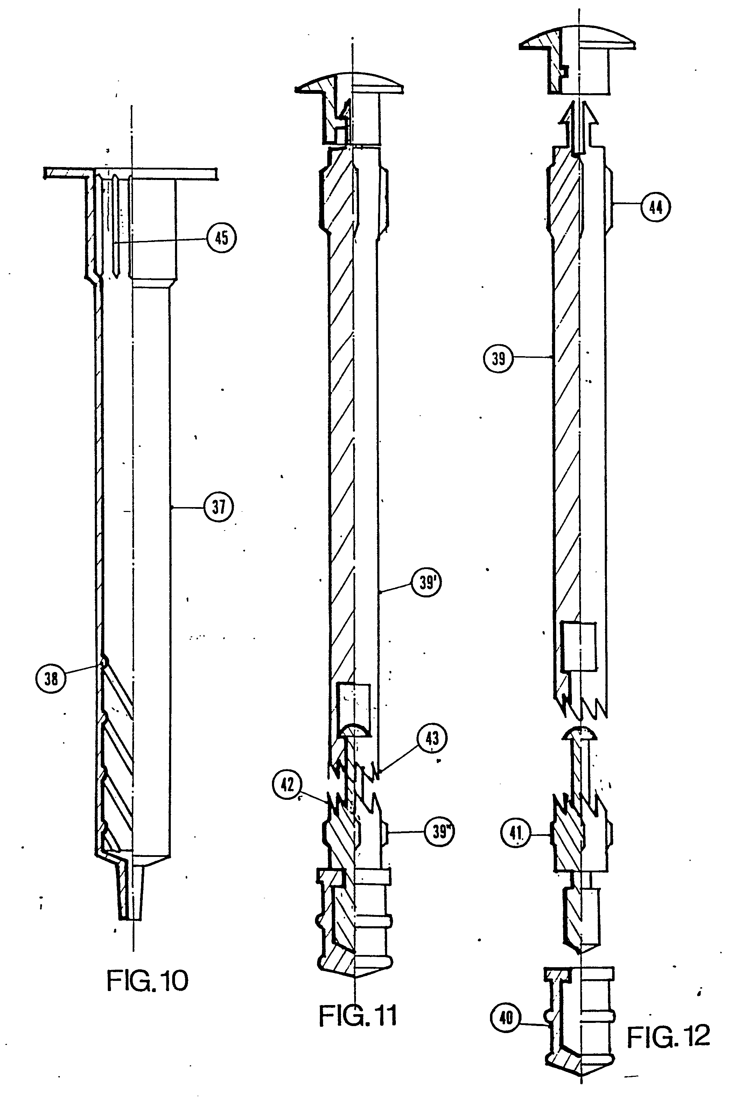

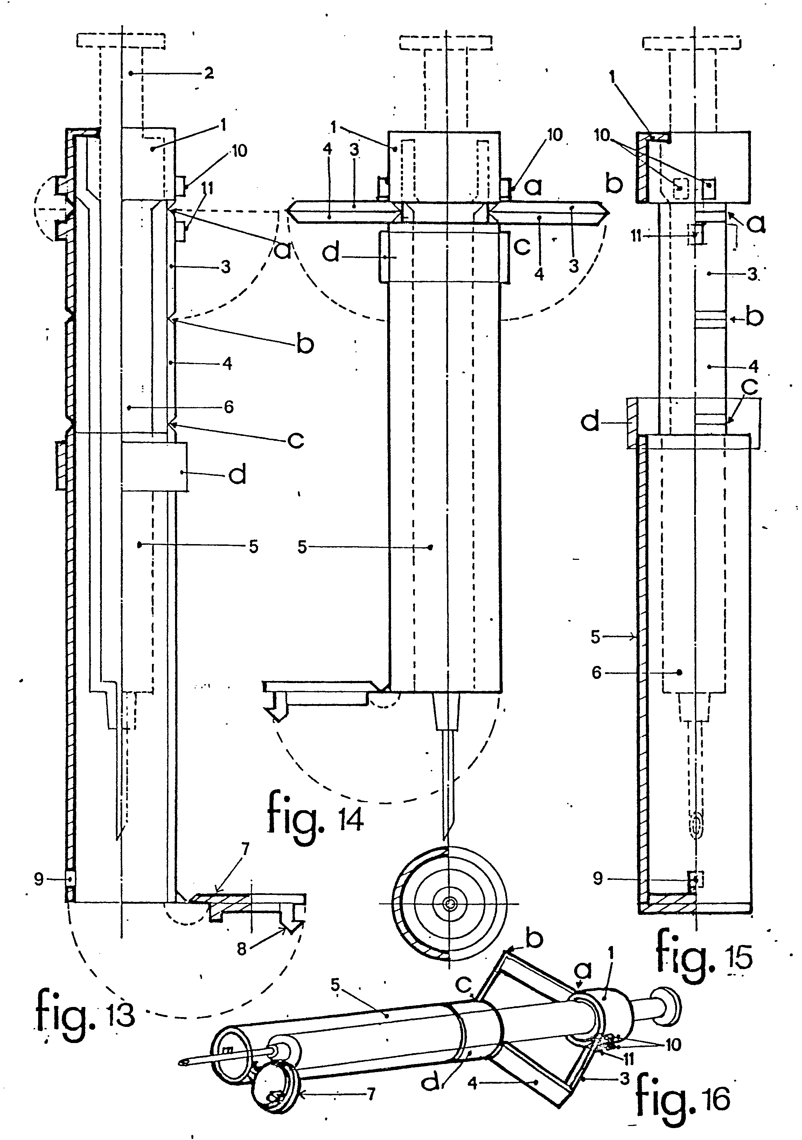

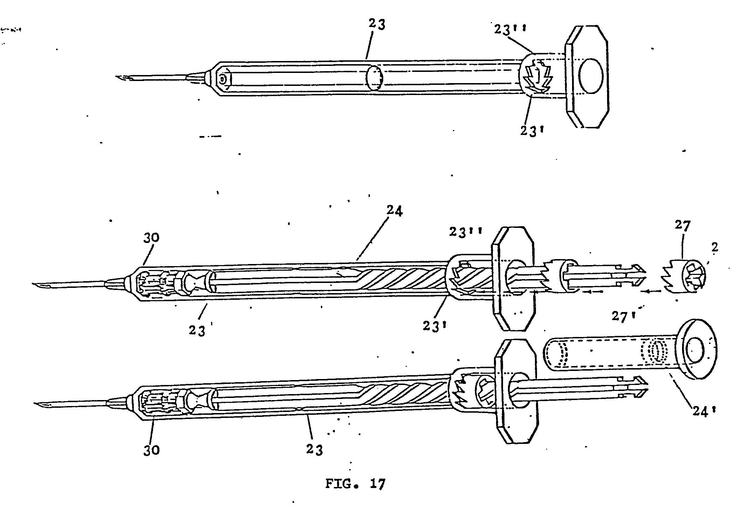

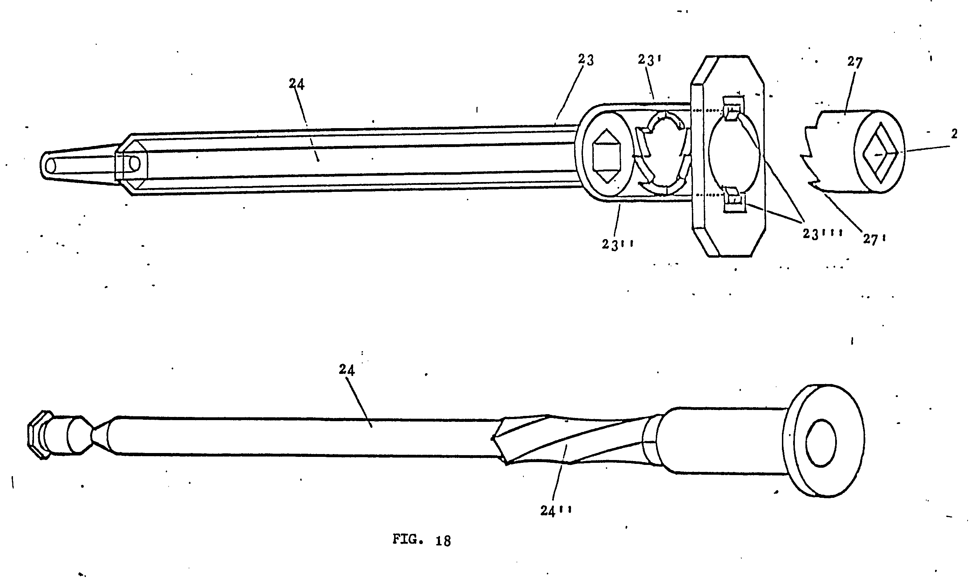

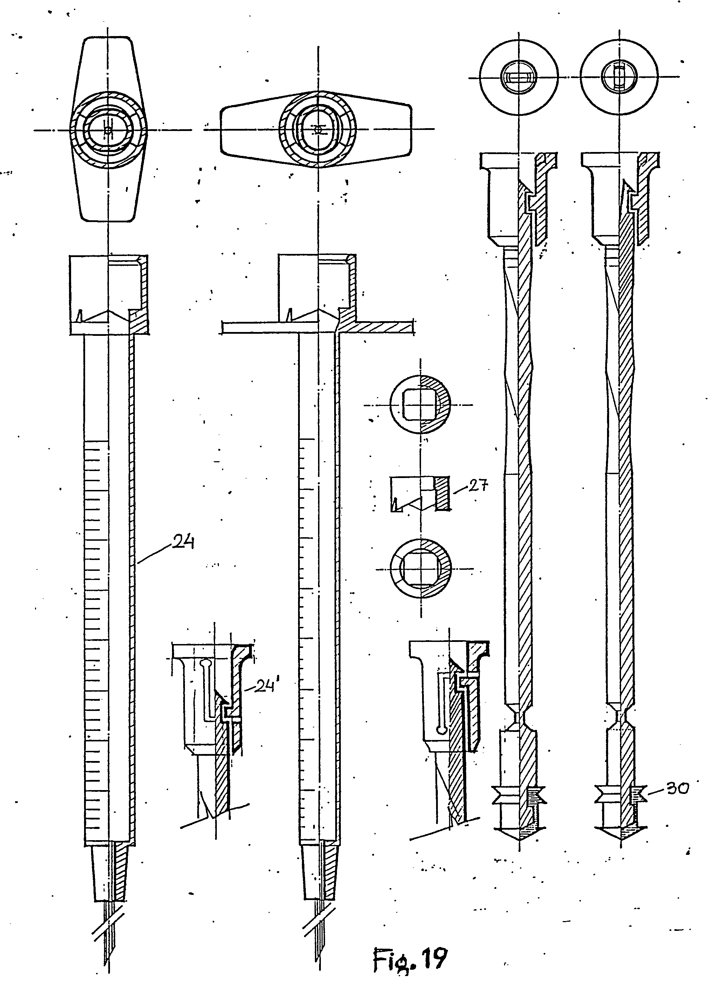

Figures 10 to 12 represent an alternative version of the syringe, which has grooves, 38, on the inside of the lower section of the outer tube of the syringe, 37 in the form of a helix-shaped thread. It also has a plunger, 39, with a plunger tip, 40, attached.

In this version, when pressure is applied to the plunger, 39, it moves downwards in a straight line and there is no rotational movement. When the plunger tip, 40, reaches the grooves, 38, innthe lower section of the outer tube of the syringe, 2, rods, 41, located on the plunger tip, slot into these grooves preventing the plunger rotating in a clockwise direction, due to the coupling or locking of the indents 42 and 43 situated on the two sections of the plunger 39´ and 39˝.

The rotation of the upper section of the plunger, however, is prevented due to the coupling of longitudinal rods, 44, on the upper section of the plunger tip, 39, which slot into longitudinal grooves, 45, on the inside upper section of the outer tube of the syringe, 37. The torque, produced in this way between the two sections of the plunger, 39´ and 39˝ causes the plunger, 39, to break at the breaking point (not illustrated) located on the plunger, 39.

In this version of the syringe, the plunger, 39, and plunger tip, 40, are locked together as described above, allowing the plunger to be pulled upwards for liquid to be drawn into the syringe prior to injection.

The shape and placing of the grooves and rods as well as the coupling of the plunger and plunger tip can be achieved using similar mechanical processes, the most important factor being to produce a torque between the two sections of the plunger so that the plunger breaks at its breaking point, situated between these sections so that when the plunger tip reaches the end of the outer tube, the syringe is rendered useless. In addition, the coupling mechanism in the form of indents or sets of teeth which disconnects while the plunger is being withdrawn prior to injection, and locks during downward movement of the plunger i.e. during the injection can be placed, in accordance with the descriptions provided, either in the upper or lower section of the plunger, the most important factor being the uncoupling of the mechanism when the plunger is withdrawn prior to injection.

Variations on the basic models of the syringe, produced by routine experimentation, would be included within the limits of the invention.

## Claims

1.- "AUTO-DESTRUCTABLE SYRINGE" for medical use with an outer tube comprising sections (2, 23, 37), a plunger tip (3, 24, 39) and a plunger (9, 24, 39) with a breaking point (18, 32) and also consisting of the following.
- A rotational mechanism (7-13; 26-28; 38-41) located in the aforementioned sections of the plunger tip and/or outer tube of the syringe, so that the movement of the said section of the plunger tip during the injection, produces the rotation of the various sections.
- A rotational block mechanism (8, 31, 41-45) to prevent the rotational movement of the plunger placed on the inside of the aforementioned section of the aforementioned section of the outer tube of the syringe and/or on the section of plunger tip and which prevents the simultaneons rotation of the two sections of the plunger on either side of the breaking point, so that as the aforementioned plunger tip is pushed further in during injection, a torque. sufficient to break the plunger at the breaking point, is produced during the final moments of the injection.
- A coupling mechanism (14-15; 34-35; 42-43) in the form of a gears or a transmission system located on either of the ends of the plunger which, when the plunger is withdrawn or pulled upwards for liquid to be draw in prior to injection, disconnects the aforementioned rotational mechanism so that neither rotation nor the resulting torque and breaking of the plunger are produced while the syringe is being prepared for injection.

2.- A syringe based on the first version but with a rotational mechanism for the plunger made up of a single component in the form of a plunger grip (10), fitted aroind the upper section of the aforementioned plunger (9), which fit into grooves (7) forming a helix on the inside of the outer tube of the syringe.

3.- Syringe based on the first and second versions with a rotational block mechanism consisting of a longitudinal rods (8) placed on the inside of the lower section of the outer tube of the syringe (2) in such a way that when the plunger (9) is pushed downwards, the plunger tip (11) becomes jammed on these rods and cannot rotate.

4.- Syringe based on versions 1,2 and 3 with a coupling mechanism consisting of indents or teeth (14, 15) or so-called 'ficam teeth' in the upper section of the plunger (9) and on the inside of the aforementioned plunger grip (10) placed around the plunger and where the plunger (9) and the plunger grip (10) are joined together by a plug system forming gears or a transmission system which allow the plunger (9) and plunger grip (10) to rotate in a clockwise direction and in unison, and separating the plunger and plunger grip when they are rotated in an anti-clockwise direction. In this way the two components are locked together while the plunger is pulled upwards to draw in liquid prior to injection.

5.- Syringe based on the second version in which the longitudinal rods (13) on the plunger grip (10) and the grooves (7) forming a helix on the inside of the inside of the syringe are designed with some movement or play.

6.- Syringe based on the first version with a rotational mechanism for the plunger consisting of a guide component (27) which fits into the upper section of the outer tube of the syringe (23) and has a opening in the shape of a cross, (28) with a thread screw in the form of a helix (26) passing through it and forms an integral part of the plunger.

7.- Syringe based on the first and sixth versions with a rotational block or brake mechanism consisting of longitudinal rods (31) on the inside of the outer tube of the syringe (23), in such a way that when the plunger (24) is pushed downwards, the plunger tip is jammed against these rods (31) thus preventing it rotating.

8.- Syringe based on the sixth and seventh versions, with a coupling mechanism consisting of indents (34-35) or "fleam teeth" located on plunger tip (40) and in the lower part of the plunger (39″)

and in which the plunger tip (40) and the plunger (39″) are locked together when the plunger is pushed downwards, when liquid is being expelled, by components which plug into one another.

9.- Syringe based on the first version with a rotational mechanism consisting of helix shaped grooves (38) on the inside of the outer tube of the syringe (37) and rods (41) on the plunger tip which slot into these grooves.

10.- Syringe based on the first and ninth versions with a rotational block or brake mechanism consisting of longitudinal grooves (45) located on the inside of the upper section of the outer tube of the syringe (37) and longitudinal rods (44) located on the upper part of the plunger (39) which slot into these grooves.

11.- Syringe based on the ninth and tenth versions with a coupling mechanism consisting of indents (42, 43) in the form of fleam teeth located on the plunger tip (40) and in the lower part of the plunger (39″), these two components being locked together when pushed downwards i.e. when liquid is being by plugs on their ends.

12.- Syringe based on the first version with a protective cylinder wich is placed over the outer tube of the syringe as a cover. This protective cylinder (4,25), is longer than the needle of the syringe so that after the injection (it would also be used as a protective cover for the needle prior to use of the syringe) the needle is covered up by sliding this cylindrical cover over the outer tube of the syringe. (4,25 and figures 3,14,15 and 16) show the needle vovered with a mechanism, held in place with articulate strips so that the needle is protcted automatically.

13.- Syringe based on the first version with a rotational block or brake mechanism consisting of a polygonal component on the inside of correspondlingly widened tube of the syringe and with a coupling mechanism consisting of indents or teeth made on the inside of the upper section of the outer tube of the syringe. Which fit into more indents or teeth located on a guide component with the plunger pusher, passing through this guide component (fig. 17, 18, 19).

14.- Syringe based on the first version, with a rotational mechanism inside the syringe wich is hollow and oval shape (not round) so that the section nearest the plunger tip fits exactly into the oval shape of the outer tube of the syringe preventing the plunger from slipping.

FIG. 1

FIG. 2

FIG. 3

FIG. 4

FIG. 5

FIG. 6

FIG. 7

FIG. 8

FIG. 9

FIG.10

FIG. 11

FIG. 12

fig. 13

fig. 14

fig. 15

fig. 16

FIG. 17

FIG. 18

24

27

24'

30

Fig. 19

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 775 364 (ALLES)<br>* Figure 7; column 2, lines 62-66 * | 1 | A 61 M 5/50<br>A 61 M 5/32 |

TECHNICAL FIELDS SEARCHED (Int. Cl.5)

A 61 M

The present search report has been drawn up for all claims

| Place of search | Date of completion of the search | Examiner |
|---|---|---|
| THE HAGUE | 19-01-1990 | SEDY, R. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)